# FASCICULE DE BREVET EUROPEEN

(11) **EP 2 729 796 B1**
(45) Date de publication et mention de la délivrance du brevet: **10.02.2016**
(21) Numéro de dépôt: 12712338.8
(22) Date de dépôt: 07.03.2012
(51) Int. Cl.: G01N 33/24, G01N 33/18, G01N 35/00, G01N 35/04

(54) **DISPOSITIF DE MESURE COUPLEE DES PARAMETRES HYDRIQUES D'UN SOL.**
MESSVORRICHTUNG ZUR GEKOPPELTEN MESSUNG VON WASSERWERTEN VON BÖDEN
DEVICE FOR COUPLED MEASUREMENT OF WATER PARAMETERS OF SOIL

(30) Priorité: 05.07.2011 FR 1156036
(43) Date de publication de la demande: 14.05.2014
(73) Titulaire: Valorhiz, 34980 Montferrier Sur Lez (FR); Institut De Recherche Pour Le Développement (IRD), 13572 Marseille Cedex 02 (FR)
(72) Inventeur: BELLIER, Gérard, F-94420 Le Plessis Trevise (FR); BRAUDEAU, Erik, F-34090 Montpellier (FR)
(74) Mandataire: Ipside
(86) Numéro de dépôt international: PCT/FR2012/050469
(87) Numéro de publication internationale: WO 2013/004927

(56) Documents cités:
- DE-C2- 19 964 265
- BRAUDEAU E. ET AL: "Hydrostructural characteristics of two African tropical soils", EUROPEAN JOURNAL OF SOIL SCIENCE, vol. 56, 1 janvier 2005 (2005-01-01), pages 375-388, XP002669933,
- BOIVIN P ET AL: "Assessment of soil compaction using soil shrinkage modelling: Experimental data and perspectives", SOIL AND TILLAGE RESEARCH, ELSEVIER, AMSTERDAM, NL, vol. 88, no. 1-2, 1 juillet 2006 (2006-07-01), pages 65-79, XP025033288, ISSN: 0167-1987, DOI: 10.1016/J.STILL.2005.04.008 [extrait le 2006-07-01]
- BRAUDEAU E ET AL: "Modeling the soil system: Bridging the gap between pedology and soil-water physics", GLOBAL AND PLANETARY CHANGE, ELSEVIER, AMSTERDAM, NL, vol. 67, no. 1-2, 1 mai 2009 (2009-05-01), pages 51-61, XP026086655, ISSN: 0921-8181, DOI: 10.1016/J.GLOPLACHA.2008.12.002 [extrait le 2009-01-03]

## Description

La présente invention appartient au domaine de la détermination des propriétés d'un milieu structuré complexe, et plus particulièrement au domaine des instruments de caractérisation physique d'échantillons poreux.

De nombreuses des publications décrivent des appareils pour l'analyse physique d'un sol en relation avec ses statuts hydriques (E. Braudeau, M. Sene et R.H. Mohtar, 2005, European Journal of Soil Science, 56: 375-388 ou P. Boivin, B. Schäffer, E. Temgoua, M. Gratier et G. Steinman, 2006, Soil & Tillage Research, 88: 65-79). L'invention a pour objet un appareil pour l'analyse physique d'un sol ou d'un autre milieu organisé naturel, qui permet de réaliser sur un même échantillon à intervalles de temps réguliers et simultanément, au moins deux mesures en relation avec l'état hydrique de cet échantillon, à savoir le potentiel de l'eau et le retrait. Les courbes obtenues à partir de ces mesures sont représentatives de l'évolution dans le temps du statut hydrique et structural de l'échantillon de sol durant son dessèchement.

Il a été démontré que la caractérisation physique précise et fiable d'un sol nécessite d'établir les deux courbes caractéristiques de l'humidité du sol, à savoir la courbe de potentiel de l'eau et la courbe de retrait (Braudeau et Mohtar, 2009, Global Planetary Change Journal, 67: 51-61). En effet, ces deux courbes sont les équations d'état de l'équilibre thermodynamique et hydrostructural du milieu organisé qu'est un sol. Elles peuvent être utilisées pour déterminer la nature du sol étudié et pour caractériser ses propriétés physiques en relation avec son utilisation agricole.

La courbe de retrait représente le volume spécifique apparent de l'échantillon (V, exprimé en dm³ par kg de sol sec) en fonction de la teneur en eau gravimétrique (notés W et exprimée en kg d'eau par kg de sol sec). La courbe de potentiel de l'eau du sol représente le potentiel de l'eau du sol (noté h et exprimé en kPa) en fonction également de la teneur en eau W.

Actuellement, la courbe de retrait est mesurée dans les conditions satisfaisantes à l'aide d'un appareillage, appelé rétractomètre à passeur d'échantillons, qui a été élaboré par le laboratoire d'hydrophysique de l'Institut de Recherche pour le Développement (IRD de Bondy). Le rétractomètre est le seul appareil actuellement capable de relever la courbe de retrait du sol en continu, c'est-à-dire à intervalles de temps réguliers au cours du dessèchement progressif et continu de l'échantillon (E. Braudeau, J.M. Costantini, G. Bellier, H. Colleuille, 1999, Soil. Sci. Soc. Am. J., 63: 525-535.).

La courbe de potentiel de l'eau est quant à elle établie en mesurant la force de succion exercée par le sol, c'est-à-dire en pratique la tension de l'eau dans un échantillon de sol non saturé. La seule méthode de mesure directe de cette grandeur physique est la mesure au tensiomètre, bien connue en sciences du sol. Ce tensiomètre comporte, relié à un capteur de dépression, un tuyau de faible diamètre rempli d'eau et muni d'une bougie poreuse à son extrémité libre. La bougie qui est plantée au coeur de l'échantillon laissant passer l'eau mais pas l'air, le système se met en équilibre de potentiel avec l'eau de l'échantillon. Le capteur de pression (par exemple une jauge de pression commune) mesure la tension de l'eau dans le tensiomètre.

Pour obtenir les courbes de retrait et de potentiel de l'eau, chacune de ces deux mesures est répétée au cours du dessèchement de l'échantillon, sur un laps de temps durant lequel l'échantillon passe de l'état humide à l'état sec. Cependant, à l'heure actuelle, elles sont réalisées indépendamment l'une de l'autre, c'est-à-dire avec un appareillage distinct, sur des échantillons différents et dans des conditions de laboratoire différentes (température et humidité de l'air). Les caractéristiques qui sont déduites des courbes obtenues, le sont en faisant l'hypothèse qu'il s'agit du même échantillon de sol et que les conditions de mesure sont identiques. Cela présente un inconvénient important, non seulement sur la durée du protocole d'obtention de ces deux mesures mais surtout sur la validité des résultats. En particulier, la référence qui est l'état saturé de l'échantillon doit être strictement identique pour les deux courbes.

Pour remédier à ces inconvénients, on souhaite réaliser la mesure de ces deux caractéristiques physiques essentielles du sol, sur un échantillon unique représentatif de la structure matricielle de l'horizon de sol dans lequel il a été prélevé. On sait en outre que les cycles d'hydratation - déshydratation modifient le comportement du sol. Il est donc nécessaire que ces mesures soient réalisées simultanément pendant un cycle unique de déshydratation. Il est également recherché que plusieurs échantillons puissent être caractérisés en parallèle, notamment lorsque l'on souhaite disposer de ces caractéristiques pour une série d'échantillons, par exemple appartenant à différents horizons d'un même profil pédologique.

C'est pour répondre à ce besoin que la présente invention a été conçue. Elle a pour but de fournir un appareil effectuant la mesure simultanée des grandeurs nécessaires à l'établissement des deux courbes caractéristiques de l'état hydrique du sol, 1) sur le même échantillon de sol, 2) durant un cycle complet de dessèchement et 3) dans les mêmes conditions standards de température et d'humidité de l'air. A ce jour aucun appareil ne permet de réaliser cette double mesure sur plusieurs échantillons à la fois.

Plus précisément, l'invention a pour objet un appareil pour l'analyse physique d'un sol en relation avec son statut hydrique selon la revendication 1.

Pour l'établissement des courbes de retrait et de potentiel de l'eau, les mesures sont répétées tout au long du processus de dessèchement progressif des échantillons, soit pendant des durées pouvant aller par exemple de 2 à 5 jours. Il est donc particulièrement intéressant de réaliser les mesures sur une séries de plusieurs échantillons. L'appareil est donc conçu pour que chacun des échantillons soit placé tout à tour dans une zone équipée des moyens de mesure, appelée zone de mesure, où sont réalisées les mesures. Ce déplacement cyclique des échantillons est réalisé essentiellement grâce à un passeur d'échantillons, dont la structure sera décrite en détail plus loin. Le passeur amène un échantillon et le dépose dans la zone de mesure, où il séjourne quelques instants, le temps que l'ensemble des moyens de mesure soit mis en oeuvre pour déterminer au temps t, les dimensions de l'échantillon, son potentiel hydrique et sa masse. Ces mesures sont répétées cycliquement, chaque passage dans la zone de mesure, et sans interruption jusqu'au dessèchement complet.

L'appareil comporte ainsi un passeur d'échantillons conçu pour permettre de réaliser les mesures sur au moins deux échantillons. Dans l'idéal cependant, on souhaite procéder aux mesures sur un plus grand nombre d'échantillons.

Ainsi, l'appareil objet de la présente invention comporte un passeur d'échantillons dont la fonction est de faire circuler les échantillons pour les amener à tour de rôle dans la zone de mesure, lequel comprend avantageusement un plateau circulaire doté d'au moins deux perçages traversants, chacun apte à recevoir en appui sur sa bordure un support d'échantillon de forme sensiblement cylindrique, ledit plateau étant mobile en rotation et en translation verticale, de sorte que lorsque le plateau s'abaisse, un des supports d'échantillon est déposé avec son échantillon dans la zone de mesure. Lorsque les mesures sont réalisées, le plateau remonte, entraînant le support d'échantillon, puis il avance d'un pas et vient placer l'échantillon suivant dans la zone de mesure, et ainsi de suite.

Les perçages traversants sont commodément circulaires, avec un diamètre légèrement supérieur à celui du socle des supports d'échantillon. Les supports d'échantillon sont suspendus, en appui libre, sur la bordure offerte par les perçages traversant. Pour ce faire, les supports peuvent être munis d'une collerette périphérique reposant sur ladite bordure. Le supports peuvent aussi offrir une partie tronconique qui vient se bloquer dans le perçage.

On souligne ici que, s'il est commode de travailler sur des échantillons cylindriques, posés sur des support de même, la présente invention peut fort bien être mise en oeuvre pour d'autres formes, qui en font également partie. C'est par soucis de clarté et de simplicité que la présente description présentera uniquement l'appareil doté de supports d'échantillon globalement cylindriques.

L'ensemble des moyens de mesure est regroupé dans la zone de mesure, c'est-à-dire à portée de l'échantillon. Les moyens de mesure de la masse des échantillons peuvent commodément être une balance, sur le plateau de laquelle le support et son échantillon seront déposés. Depuis cet emplacement les autres mesures seront réalisées.

Les dimensions de l'échantillon peuvent être relevées par différentes méthodes connues de l'homme de l'art (par exemple par usage de capteurs d'élongation, de proximité, etc...).

L'invention propose une méthode optique de mesure sans contact avec l'échantillon , par laquelle le diamètre et la hauteur de l'échantillon sont mesurés. Le volume est ensuite calculé, son évolution dans le temps correspondant à la valeur du retrait. Les organes émetteurs et récepteurs du système optique sont placés à proximité de l'échantillon, dans la zone de mesure.

Enfin, la tension de l'eau peut être mesurée par un tensiomètre, d'un modèle disponible dans le commerce ou dans les laboratoires, tel que le mini-tensiomètre à bougie poreuse de 2 mm de diamètre par exemple. Ce tensiomètre doit être placé dans la zone de mesure pour être à même de relever la valeur de la tension de l'eau simultanément avec la prise des autres mesures. Selon l'invention, il est placé à l'intérieur du support d'échantillon, sous l'échantillon lui-même.

Ainsi, selon l'invention, l'appareil comprend :
- une balance dotée d'un plateau apte à recevoir et à peser le support d'échantillon et son échantillon,
- un système optique de mesure de la hauteur et du diamètre dudit échantillon lorsque celui-ci est placé sur ledit plateau de balance, et
- pour chaque échantillon, un tensiomètre dit "à bougie poreuse" apte à mesurer le potentiel de l'eau dudit échantillon, comprenant un tube souple fermé par une bougie en céramique perméable à une extrémité et un manomètre à l'autre, ledit manomètre étant logé dans le support dudit échantillon.

Le principe du tensiomètre à bougie poreuse est bien connu dans le domaine des sciences du sol. La bougie poreuse est enfoncée au centre de l'échantillon. Un tube capillaire, généralement en matière plastique souple, la relie à un manomètre. On désigne par manomètre l'organe du tensiomètre qui est sensible aux variations de pression du fluide dans un espace fermé. Différents types de manomètres existent et peuvent être mis en oeuvre dans la présente invention. On peut par exemple utiliser un capteur de pression à membrane placé dans une chambre en relation avec le tube capillaire. La déformation de la membrane est proportionnelle à la pression exercée dans la chambre par le fluide. La mesure de la déformation, effectuée pour des pressions connues, permet d'obtenir des tables de référence.

Selon l'invention, le manomètre est logé dans le support dudit échantillon. En d'autres termes, chaque support d'échantillon accueille un manomètre dont la bougie poreuse est fichée dans l'échantillon qu'il porte. Ainsi, quand le support est placé avec son échantillon dans la zone de mesure, la pression traduisant le potentiel de l'eau de l'échantillon peut être relevée, en même temps que celui-ci est pesé et mesuré en hauteur et diamètre.

L'association du tensiomètre avec le support d'échantillon peut être réalisée de différentes manières. Selon un mode avantageux de réalisation de l'appareil objet de la présente invention, chaque support d'échantillon comprend :
- un socle sensiblement cylindrique surmonté d'une plaque horizontale destinée à soutenir un échantillon, et
- un bloc manométrique fixé à la paroi dudit socle, et dans lequel est ménagée une chambre hermétique communiquant avec ladite bougie poreuse par l'intermédiaire dudit tube souple, ladite chambre étant associée à un capteur de pression à membrane.

La plaque horizontale peut être fixée au socle par des entretoises, laissant un espace entre la plaque et le socle. Ceci favorise l'évaporation homogène de l'eau de l'échantillon, d'autant que l'on peut avantageusement ménager des perforations dans ladite plaque. On met à profit l'espace libre pour faire passer le tube capillaire de la chambre vers l'échantillon au-dessus.

Le bloc manométrique peut être fixé à la paroi du socle, de préférence dans sa partie supérieure, par tout moyen connu de l'homme de l'art. Il peut notamment être vissé à la paroi du socle, ou bien être maintenu par un épaulement ménagé sur son pourtour coopérant avec une rainure complémentaire du socle, ou autre.

Il peut comporter un évidemment formant une chambre, dans laquelle est placé le capteur de pression. Selon un mode de réalisation particulier, le capteur peut être emboîté à la base de la chambre, de sorte que la chambre est fermée par le capteur. Dans ce cas, un joint torique peut assurer avantageusement l'étanchéité. Ainsi, selon une caractéristique préférée de l'invention, chaque capteur constitue un tampon d'obturation de la chambre.

Selon une autre caractéristique préférée de l'invention, chaque chambre comporte un plafond bombé au sommet duquel débouche un conduit de remplissage muni d'une vanne. Avant de la mise en route de l'appareil, les échantillons sont posés sur la plaque horizontale, les tensiomètres à bougie poreuse préalablement remplis d'eau dégazée sont enfoncés par le côté jusqu'au coeur des échantillons puis branchés sur les blocs manométriques qui auront également été remplis d'eau en utilisant le conduit de remplissage terminé par une vanne. Cette opération doit permettre d'obtenir un système ne contenant que de l'eau dégazée. Grâce à la géométrie de la chambre et de son conduit d'entrée, on évite la formation de bulles d'air lors du remplissage de la chambre.

Lorsqu'un support d'échantillon est déposé sur le plateau de la balance, les mesures doivent démarrer. En particulier, les valeurs perçues par le manomètre doivent être transmises à l'opérateur ou enregistrées pour traitement ultérieur. Il n'est pas nécessaire que ces valeurs soient lues en permanence, il suffit qu'elles soient relevées en même temps que les autres grandeurs (masse et dimensions).

C'est pourquoi, dans un mode de réalisation particulier de l'appareil selon l'invention, chaque manomètre est relié à des contacteurs électriques maintenus à proximité de la base du socle, et le plateau de la balance est muni d'une platine dotée de plots électriques, les contacteurs étant disposés de manière à entrer en contact avec les plots lorsque le support d'échantillon est placé sur la balance. Une tringle peut être disposée dans le socle pour maintenir les contacteurs dans la position adéquate.

Les mesures sont réalisés pendant plusieurs heures, voire plusieurs jours, durant lesquels les échantillons se dessèchent progressivement, l'eau s'évaporant et libérant la porosité pour être remplacée par l'air atmosphérique. Il est décisif que les échanges eau-air soient les plus homogènes possible dans toute l'épaisseur de l'échantillon et de tous côtés. On a vu que la plaque horizontale peut être fixée au socle par des entretoises, laissant un espace entre la plaque et le socle. On améliore encore les échanges au niveau de la plaque par des perforations, en nombre important bien que de taille modérée, afin que l'échantillon ne se déstructure pas à cause d'une évaporation qui ne serait pas homogène tout autour de l'échantillon.

C'est pourquoi la plaque de chaque support d'échantillon est de préférence dotée de perforations autorisant le passage d'eau et d'air entre l'atmosphère et la base de l'échantillon.

Comme indiqué plus haut, la mesure de la taille de l'échantillon placé dans la zone de mesure se fait selon un système optique. On a commodément recours à une mesure par deux sous-systèmes, l'un dédié à la mesure du diamètre de l'échantillon (pour autant qu'il est cylindrique), l'autre dédié à la mesure de sa hauteur.

Dans un aspect particulier de l'appareil selon l'invention, le système optique de mesure du diamètre dudit échantillon comprend deux capteurs à barrière laser placés dans la zone de mesure de part et d'autre de l'échantillon lorsque celui-ci se trouve sur la balance, à une distance mutuelle inférieure au diamètre minimum supposé des échantillons.

D'une manière générale, le principe est que l'objet mesuré intercepte partiellement le faisceau laser. La taille de l'ombre, proportionnelle au diamètre de l'objet, correspond à une certaine quantité de lumière interceptée. La réduction du diamètre se traduit par un changement de la quantité de lumière reçue (voltage). On peut utiliser deux capteurs de type photoélectriques à barrière laser. Ils permettent de déterminer à chaque passage le diamètre de l'échantillon de sol tout au long de son retrait dû au dessèchement. Il seront placés l'un par rapport à l'autre à une distance telle que l'échantillon coupe les deux faisceaux même en fin de processus. Pour ce faire, ils peuvent être montés sur une tige horizontale, de sorte que leur écartement peut être réglé convenablement en début de cycle. Cette tige permet aussi de régler correctement la distance entre l'émetteur et le récepteur de chaque capteur.

Selon un autre aspect de l'appareil objet de l'invention, le système optique de mesure de la hauteur dudit échantillon comprend un capteur laser sans contact placé à l'aplomb de la zone de mesure, apte à émettre un faisceau lumineux vers une pastille posée à plat sur l'échantillon et à détecter le faisceau réfléchi pour déterminer par triangulation l'éloignement de ladite pastille. D'une manière générale, ce type de capteur calcule la distance qui sépare un objet à mesurer d'un récepteur (détecteur photodiode). Dans notre cas, la cible sera une pastille métallique posée à plat sur l'échantillon. Ce capteur permet donc de déterminer la variation de la hauteur de l'échantillon de sol tout au long de son retrait. Il peut être monté sur une tige verticale, de sorte que sa position peut être réglée convenablement en début de cycle.

Selon une caractéristique particulièrement intéressante de la présente invention, la balance sur laquelle le support et son échantillon sont déposées comprend un dispositif de compensation de hauteur en fonction de la masse déposée sur son plateau. En effet, on comprend que lorsque l'eau est remplacée par de l'air dans la porosité de l'échantillon, la masse de celui-ci change, et par conséquent le plateau de la balance est soumis à une force moindre à chaque cycle. La valeur relevée par le capteur à l'aplomb de l'échantillon peut donc en être affectée. Il est possible d'introduire un biais dans le traitement des relevés de mesures pour tenir compte de cette perte de masse et du fait que le plateau descend de moins en moins bas, mais il a été choisi pour l'invention de mettre en oeuvre un moyen de garder le plateau fixe, quelle que soit la masse de l'objet pesé. On utilise donc de préférence une balance à compensation, qui permet de faire des mesures sans déplacement du plateau, au centigramme près.

Il peut être commode de s'assurer que les supports d'échantillon sont correctement orientés. Selon une caractéristique intéressante de l'invention, l'appareil peut comprendre un dispositif de positionnement, afin que les supports et les échantillons soient correctement placés, au début et tout au long de la mesure. Il peut être constitué d'une encoche ménagée dans les supports d'échantillon coopérant avec un pointeau ménagé sur la bordure des perçages du plateau circulaire. On fera ainsi coïncider les plots électriques de la balance et les contacteurs du manomètre. On placera aussi les tubes capillaires reliant la chambre manométrique à la bougie poreuse vers le centre du plateau, hors du champs des faisceaux lasers pour ne pas perturber la mesure optique.

Les organes décrits ci-dessus sont assemblés pour fonctionner ensemble, et pour déterminer les caractéristiques physiques de plusieurs échantillons dans une même opération. Il va sans dire que, au moins au regard de sa durée, le processus doit être robotisé et automatisé.

Ainsi, de préférence selon l'invention, le passeur d'échantillons comprend un plateau circulaire comportant huit perçages traversants, et une colonne de levage actionnée par un premier moteur assurant le déplacement vertical du plateau et par un second moteur assurant la rotation dudit plateau circulaire.

L'appareil selon l'invention peut comprendre en outre :
- une étuve thermostatée apte à contenir au moins le passeur d'échantillons et l'ensemble des moyens de mesure,
- des moyens de pilotage du passeur d'échantillons et des moyens de mesure,
- des moyens de réception et de traitement des signaux mesurés, pour obtenir des données de mesures quantitatives,
- des moyens d'enregistrement des données, de préférence au fur et à mesure de leur acquisition.

Les données sont au moins : le temps, le diamètre, la hauteur, la masse et la dépression dans le tensiomètre.

Selon un aspect particulièrement intéressant de l'invention, l'appareil comprend des moyens de traitement des données enregistrées pour chacun des échantillons au cours d'un unique cycle d'évaporation depuis l'état saturé jusqu'à l'état sec, et des moyens de calcul de la courbe de retrait et de la courbe du potentiel de l'eau desdits échantillons. Sont calculées la courbe de retrait V = f(W) et la courbe de potentiel h = f(W). Ces courbes pourront ensuite être elles-mêmes traitées à l'aide de modèles préétablis, en vue de caractériser la structure du sol auquel l'échantillon appartient, ou plus généralement la structure du milieu dans lequel l'échantillon a été prélevé.

La présente invention trouve de nombreuses applications, notamment pour la modélisation des relations physiques BIO-SOL-CLIMAT en sciences agro-environnementales, mais aussi pour établir une typologie des sols (bases de données sol, cartographie pédologique) utilisables par les laboratoires de biologie et d'écologie des sols.

Le secteur industriel travaillant sur la mise au point de produits nouveaux en direction de l'agriculture et de l'environnement, dispose désormais d'un outil pour tester et caractériser le comportement ou le devenir de ces produits dans le sol, en tenant compte du type de sol sous une variabilité climatique donnée. Il est alors possible de prévoir l'impact sur le long terme de ces produits qui tienne compte de la variété des sols et de leurs fonctionnement hydrostructural et pédoclimatiques.

L'appareil de mesure couplée des deux courbes caractéristiques d'humidité du sol, la courbe de retrait et la courbe du potentiel de l'eau, est ainsi au coeur d'une nouvelle méthodologie de caractérisation et modélisation hydrostructurale des sols.

La présente invention sera mieux comprise, et des détails en relevant apparaîtront, grâce à la description qui va être faite d'une de ses variantes de réalisation, en relation avec les figures annexées, dans lesquelles :
La fig.1 est une vue d'ensemble en perspective de l'appareil selon l'invention.
Les fig. 2a et 2b représentent la zone de mesure de l'appareil selon l'invention, avant (2a) et après (2b) dépose de l'échantillon sur le plateau de la balance.

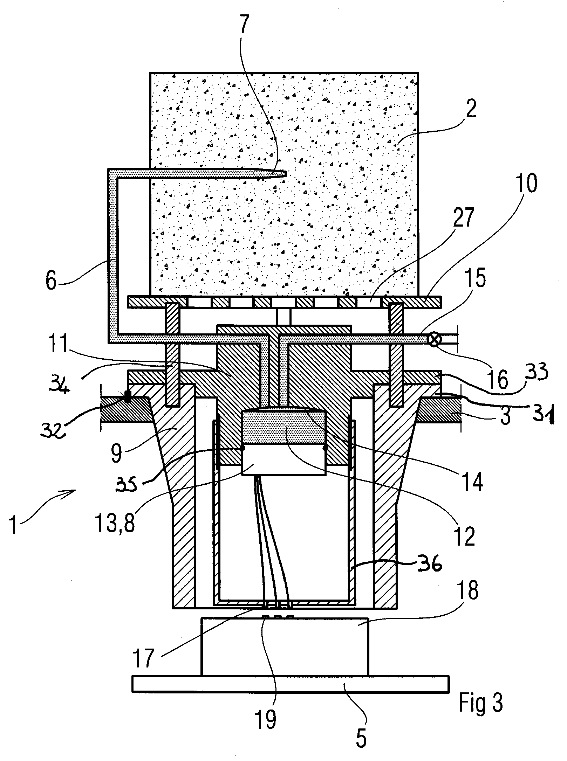

La fig. 3 est une vue en coupe d'un support d'échantillon selon l'invention, avec son échantillon et son système de mesure de la tension de l'eau.
Les fig. 4 et 5 sont des représentations schématiques des systèmes de mesure optiques.
La fig. 6 est la courbe de retrait d'un des huit échantillons obtenue à l'aide de l'appareil selon l'invention
La fig. 7 est la courbe de potentiel de l'eau (ou de succion) du même échantillon , obtenue à l'aide de l'appareil selon l'invention, simultanément.

### EXEMPLE 1

### Appareil de mesure couplée

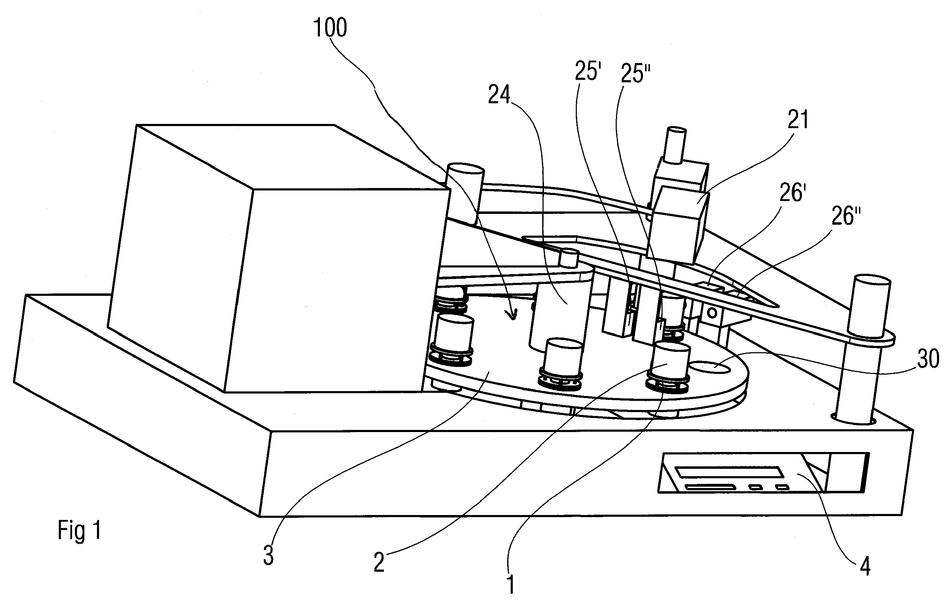

Sur la fig.1, est représenté un appareil pour la détermination quantitative des caractéristiques physiques d'un sol selon l'invention. Il comprend le passeur d'échantillons 100, qui peut recevoir huit supports d'échantillon 1. Le passeur 100 comprend le plateau circulaire 3 dans lequel on a huit perçages traversants 30. Il est associé à une colonne de levage 24 actionnée par un moteur assurant le déplacement vertical du plateau circulaire 3. Un second moteur assure la rotation du plateau circulaire. Ces passeurs d'échantillons sont connus, voir DE 199 64 265 C 2.

Il comprend également des moyens de mesure des dimensions desdits échantillons, des moyens de mesure du potentiel de l'eau desdits échantillons, et des moyens de mesure de la masse desdits échantillons. Ces moyens sont disposés de manière à définir une zone de mesure, dans laquelle les échantillons 2 sont placés pour être soumis aux dites mesures.

Les échantillons sont typiquement des carottes cylindriques de sol, prélevées en milieu naturel. Ils sont saturés en eau avant d'être placés sur les supports et soumis aux mesures.

Le passeur d'échantillons 100 est doté de moyens pour placer chaque support d'échantillon 1 et son échantillon 2 dans la zone de mesure tour à tour et selon un cycle répété dans le temps. Il comprend le plateau circulaire 3 doté de huit perçages traversants 30. Chaque perçage 30 reçoit un support d'échantillon 1, qui repose en appui sur sa bordure. Les perçages traversants sont circulaires, avec un diamètre légèrement supérieur à celui du socle 9 des supports d'échantillon 1 (qui seront décrits plus loin).

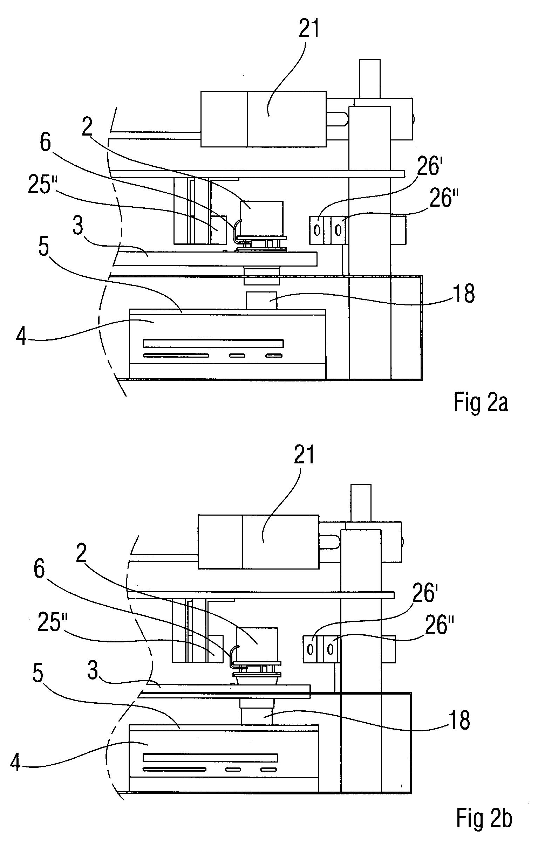

Le plateau circulaire 3 est mobile en rotation et en translation verticale, de sorte que lorsqu'il s'abaisse, un des supports d'échantillon 1 est déposé avec son échantillon 2 dans la zone de mesure, comme illustré sur les figures 2a et 2b. On note que la partie supérieure du socle 9 des supports 1 comporte la collerette 31 qui repose sur la bordure du perçage 30 du plateau circulaire 3.

Les moyens de mesure de la masse des échantillons 2 sont constitués essentiellement d'une balance 4 (balance électronique au cg, de portée maximale 1600 g - modèle Mettler - Toledo PB 1502), sur le plateau 5 de laquelle un support 1 et son échantillon 2 seront déposés. La balance 4 est dite "à plateau fixe", car elle comprend un dispositif de compensation de la hauteur du plateau en fonction de la masse déposée sur celui-ci.

Le plateau de la balance est muni de la platine 18 qui permet d'établir une liaison électrique provisoire entre le support d'échantillon qui est posé dessus, et les organes de réception et de traitement des données mesurées. Pour ce faire, la platine 18 est dotée des plots électriques 19, qui ont pour rôle d'établir la liaison avec des contacteurs 17 portés par les supports d'échantillon. Les plots 19 seront donc en contact successivement et cycliquement avec les contacteurs 17 des différents supports d'échantillon au fil des relevés de mesures.

La tension de l'eau est mesurée par un tensiomètre à bougie poreuse. Il comprend le tube capillaire 6 en matière plastique souple, fermé par la bougie 7 en céramique perméable, qui est enfoncée au coeur de l'échantillon. L'autre extrémité du tube souple 6 est reliée au manomètre 8, qui est logé dans le support d'échantillon 1, sous l'échantillon lui-même, de sorte qu'il permet de relever la valeur de la tension de l'eau simultanément avec la prise des autres mesures.

Comme illustré sur la figure 2, l'association du tensiomètre et du support d'échantillon est réalisée par l'intermédiaire d'un élément de soutien, appelé bloc manométrique, incorporant l'organe sensible du tensiomètre, dans le cas présent un capteur de pression à membrane. Chaque support d'échantillon 1 comprend le socle 9 de section cylindrique, surmonté de la plaque horizontale 10 destinée à soutenir un échantillon. La plaque horizontale 10 est percée des perforations 27. Elle est fixée au socle 9 par les entretoises 34, laissant un espace entre la plaque et le socle.

Le bloc manométrique 11 est fixé à la paroi du socle 9, sous la plaque 10, par l'épaulement 33 ménagé sur son pourtour qui est assemblé sur la collerette 31 du socle.

Le bloc manométrique 11 comporte un évidemment constituant la chambre hermétique 12, dans laquelle est placé le capteur de pression 13. La chambre 12 communique avec la bougie poreuse 7 par l'intermédiaire du tube souple 6. Le capteur 13 est emboîté à la base de la chambre 12, de sorte que la chambre est fermée par le capteur. Le joint torique 35 assure l'étanchéité de l'assemblage. Le plafond 14 de la chambre 12 est bombé afin que des bulles d'air ne soient pas piégées. A son sommet, débouche le conduit de remplissage 15, muni de la vanne 16.

Le capteur 13 est relié aux contacteurs électriques 17 maintenus à proximité de la base du socle 9. Ces contacteurs sont disposés de manière à entrer en contact avec les plots électriques 19 équipant le plateau 5 de la balance 4, lorsque le support d'échantillon 1 y est déposé. La tringle 36 est disposée dans le socle 9 pour maintenir les contacteurs 17 dans la position adéquate.

Lorsqu'un support d'échantillon 1 est déposé sur le plateau 3 de la balance 4, les mesures démarrent. Les valeurs perçues par le manomètre 8 sont transmises à l'opérateur ou enregistrées pour traitement ultérieur.

Enfin, les dimensions de l'échantillon sont relevées par une méthode optique, par deux sous-systèmes, l'un dédié à la mesure du diamètre de l'échantillon, l'autre dédié à la mesure de sa hauteur.

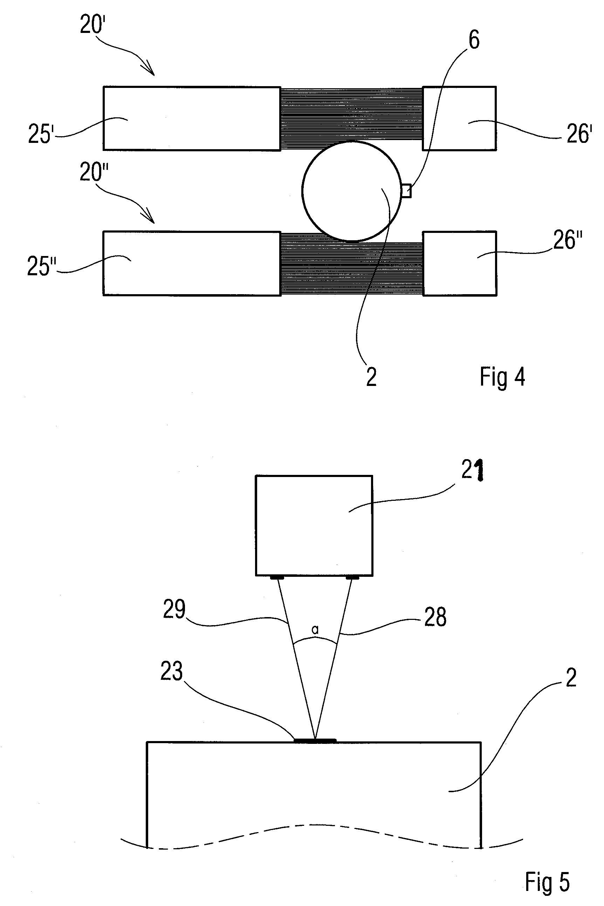

Comme illustré sur la figure 4, le système optique de mesure du diamètre de l'échantillon comprend deux capteurs à barrière laser 20' et 20" (capteurs photoélectriques à barrières lasers de type Keyence LX2-70). La source 25' et 25" et le récepteur 26' et 26" de chaque capteur sont placés en vis-à-vis de part et d'autre de la zone de mesure, de sorte que l'échantillon coupe le faisceau quand il est placé dans la zone de mesure. Les capteurs 20' et 20" sont montés sur des tiges horizontales (non représentées) et leur distance mutuelle peut être réglée par coulissement sur la tige, de manière à ce qu'elle soit inférieure au diamètre minimum qu'auront les échantillons totalement desséchés. Comme illustré sur la figure 5, le système optique de mesure de la hauteur de l'échantillon 2 comprend le capteur laser sans contact 21 (type Keyence LB72), placé à l'aplomb de la zone de mesure. Il émet un faisceau lumineux 28 vers la pastille métallique 23 posée à plat sur l'échantillon 2 et il détecte le faisceau réfléchi 29. Puis, l'éloignement de la pastille 23 est déterminé par la méthode dite de triangulation. Le capteur 21 est monté sur une tige verticale (non représentée) de sorte que sa position peut être réglée convenablement en début de cycle.

Ainsi, l'ensemble desdits moyens de mesure sont regroupés dans la zone de mesure pour être mis en oeuvre simultanément sur un échantillon donné lorsqu'il est placé dans ladite zone de mesure. Afin que les supports 1 et les échantillons 2 soient correctement placés au début et tout au long des mesures, une encoche est ménagée dans les supports d'échantillon. Elle coopère avec un pointeau 32 ménagé sur la bordure de chaque perçage 30 du plateau circulaire 3.

Les moyens et organes qui viennent d'être décrits sont agencés pour former l'appareil selon l'invention, qui peut comporter d'autres éléments communément utilisés pour le bon fonctionnement des équipements de mesure, tel qu'un capot, des pieds réglables en hauteur, ou autres. Il comprend également une étuve thermostatée d'un volume suffisant pour contenir le passeur d'échantillons et l'ensemble des moyens de mesure.

L'appareil selon l'exemple est associé à des moyens de pilotage du passeur d'échantillons et des moyens de mesure. Il est assisté de moyens de réception et de traitement des signaux, pour obtenir des données de mesures quantitatives, ainsi qu'à des moyens d'enregistrement des données, de préférence au fur et à mesure de leur acquisition.

### EXEMPLE 2

### Mise en oeuvre de l'appareil sur une série de huit échantillons

Des échantillons de sol de 100 cm3 environ ont été prélevés dans huit sites naturels, pour obtenir huit cylindres de dimension identiques, représentatifs de la structure matricielle de l'horizon de sol dans lequel ils ont été prélevés. Les cylindres ont une taille variant entre 50 cm et 66 cm de diamètre, et entre 50 cm et 30 cm de hauteur. La taille est choisie à bon escient selon la texture plus ou moins argileuse ou sableuse présidant à la stabilité de la structure, son homogénéité, et au temps que demandera le dessèchement.

Après saturation en eau par infiltration *per ascensum* de l'échantillon posé sur une lame d'eau pendant au moins 4 heures, les huit échantillons sont placés sur chacun des supports de l'appareil décrit à l'exemple 1. La bougie poreuse est insérée dans chaque échantillon, la chambre du manomètre est remplie d'eau. Les supports ainsi équipés sont placés dans les perçages du passeur d'échantillon, dans l'étuve thermostatée à 30 °C, de sorte que les échantillons se dessèchent régulièrement par évaporation de l'eau à température constante, depuis l'état saturé jusqu'à l'état sec.

L'appareil est mis en route. Il effectue sur la série des huit échantillons à intervalles réguliers d'environ 10 minutes entre chaque passage du même échantillon, pendant une durée d'au moins 2 jours dépendant de la capacité en eau de l'échantillon, la mesure des quatre variables suivantes : le diamètre et la hauteur de l'échantillon, son poids, et la tension de l'eau mesurée au tensiomètre.

A chaque passage d'un échantillon, le plateau descend et dépose le porte échantillon devant les capteurs laser, sur le plateau de la balance, sur lequel un réceptacle met en contact les plots d'entrées sorties de courant du capteur de pression avec l'automate de pilotage de l'appareil et de saisie des données.

Les données de mesure sont enregistrées au fur et à mesure de l'avancement. Les données recueillies sont : le temps, le diamètre, la hauteur, le poids et la dépression dans le tensiomètre (de 0 à 80 kPa). Ces données sont complétées par quelques mesures de référence faites à la fin de l'expérience (mesure du poids sec des échantillons portés à l'étuve à 105°C, mesure du volume apparent sec) pour que les courbes de retrait, V=f(W) et de potentiel, h=f(W), puissent être calculées.

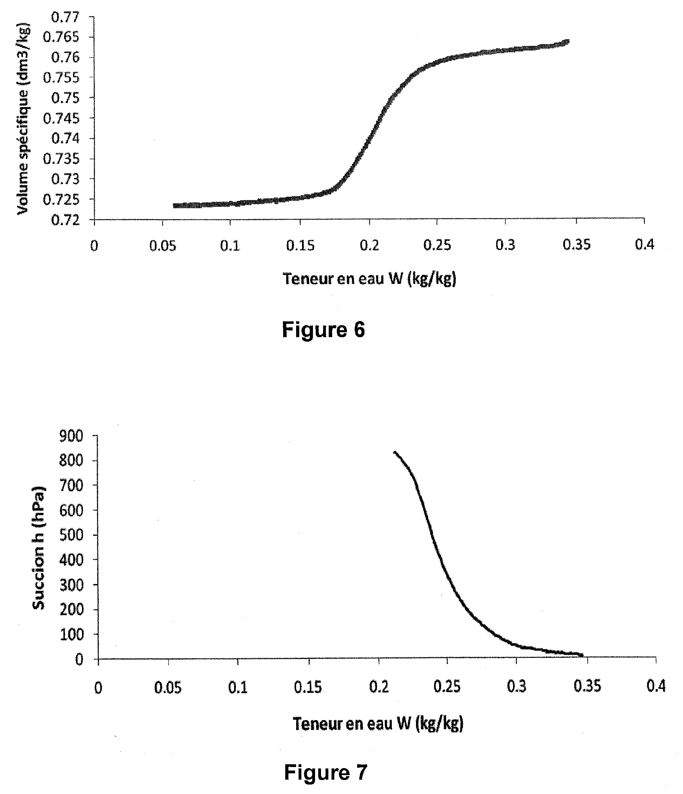

La courbe de retrait et la courbe de potentiel de l'eau sont tracées : voir fig 6 et 7. On en déduit le volume spécifique V, la teneur en eau W et le potentiel de l'eau h au temps t entre l'état humide et l'état sec.

Le traitement informatisé des deux courbes considérées ensemble permettra d'extraire les paramètres hydrostructuraux de la pédostructure des sols analysés.

## Revendications

1. Appareil pour l'analyse physique d'un sol en relation avec son statut hydrique, comportant un passeur d'échantillons (100) apte à recevoir au moins deux supports (1) d'échantillons de sol, ledit passeur d'échantillons étant doté de moyens pour placer chaque support d'échantillon et son échantillon (2) dans une zone de mesure tour à tour et selon un cycle répété dans le temps, **caractérisé en ce qu'**il comprend :
- des moyens de mesure de la masse desdits échantillons, comportant une balance (4) dotée d'un plateau (5), apte à recevoir et à peser le support d'échantillon (1) et son échantillon (2) ;
- des moyens de mesure des dimensions desdits échantillons, comportant un système optique de mesure de la hauteur et du diamètre dudit échantillon lorsque celui-ci est placé sur ledit plateau de balance ; et,
- des moyens de mesure du potentiel de l'eau desdits échantillons, comportant pour chaque échantillon, un tensiomètre dit « à bougie poreuse » apte à mesurer le potentiel de l'eau dudit échantillon et comprenant un tube (6) souple fermé par une bougie (7) en céramique perméable à une extrémité et un manomètre (8) à l'autre, ledit manomètre étant logé dans le support dudit échantillon ;
l'ensemble desdits moyens de mesure étant regroupés dans ladite zone de mesure pour être mis en oeuvre simultanément sur un échantillon donné lorsqu'il est placé dans ladite zone de mesure.

2. Appareil selon la revendication 1, **caractérisé en ce que** le passeur d'échantillons comprend un plateau circulaire (3) doté d'au moins deux perçages traversants (30), chacun apte à recevoir en appui sur sa bordure un support d'échantillon (1) de forme sensiblement cylindrique, ledit plateau étant mobile en rotation et en translation verticale, de sorte que lorsque le plateau s'abaisse, un des supports d'échantillon (1) est déposé sur la balance (4) avec son échantillon (2) dans la zone de mesure.

3. Appareil selon l'une quelconque des revendications 1 ou 2, **caractérisé en ce que** chaque support d'échantillon (1) comprend :
- un socle (9) sensiblement cylindrique surmonté d'une plaque (10) horizontale destinée à soutenir un échantillon (2), et
- un bloc manométrique (11) fixé à la paroi dudit socle, et dans lequel est ménagée une chambre hermétique (12) communiquant avec la bougie poreuse (7) par l'intermédiaire du tube souple (6), ladite chambre étant associée à un capteur de pression à membrane (13).

4. Appareil selon la revendication précédente, **caractérisé en ce que** chaque capteur (13) constitue un tampon d'obturation de la chambre (12).

5. Appareil selon l'une quelconque des revendications 3 ou 4, **caractérisé en ce que** chaque chambre (12) comporte un plafond (14) bombé au sommet duquel débouche un conduit de remplissage (15) muni d'une vanne (16).

6. Appareil selon l'une quelconque des revendications 1 à 5, **caractérisé en ce que** chaque manomètre (8) est relié à des contacteurs électriques (17) maintenus à proximité de la base du socle (9), et le plateau (5) de la balance (4) est muni d'une platine (18) dotée de plots électriques (19), lesdits contacteurs étant disposés de manière à entrer en contact avec lesdits plots lorsque le support d'échantillon (1) est placé sur la balance.

7. Appareil selon l'une quelconque des revendications 3 à 6, **caractérisé en ce que** la plaque (10) de chaque support d'échantillon (1) est dotée de perforations autorisant le passage d'eau et d'air entre l'atmosphère et la base de l'échantillon (2).

8. Appareil selon l'une quelconque des revendications 1 à 7, **caractérisé en ce que** le système optique de mesure du diamètre de l'échantillon (2) comprend deux capteurs à barrière laser (20', 20") placés dans la zone de mesure de part et d'autre dudit échantillon lorsque celui-ci est placé sur la balance (4), à une distance mutuelle inférieure au diamètre minimum supposé des échantillons.

9. Appareil selon l'une quelconque des revendications 1 à 8, **caractérisé en ce que** le système optique de mesure de la hauteur de l'échantillon (2) comprend un capteur laser sans contact (21) placé à l'aplomb de la zone de mesure, apte à émettre un faisceau lumineux vers une pastille (23) posée à plat sur ledit échantillon et à détecter le faisceau réfléchi pour déterminer par triangulation l'éloignement de ladite pastille.

10. Appareil selon l'une quelconque des revendications 1 à 9, **caractérisé en ce que** la balance (4) comprend un dispositif de compensation de hauteur en fonction de la masse déposée sur son plateau (5).

11. Appareil selon l'une quelconque des revendications précédentes, **caractérisé en ce que** le passeur d'échantillons (100) comprend un plateau circulaire (3) comportant huit perçages traversants (30), et une colonne de levage (24) actionnée par un premier moteur assurant le déplacement vertical dudit plateau circulaire, et par un second moteur assurant la rotation dudit plateau circulaire.

12. Appareil selon l'une quelconque des revendications précédentes, **caractérisé en ce qu'**il comprend en outre :
- une étuve thermostatée apte à contenir au moins le passeur d'échantillons et l'ensemble des moyens de mesure,
- des moyens de pilotage du passeur d'échantillon et des moyens de mesure,
- des moyens de réception et de traitement des signaux mesurés pour obtenir des données de mesures quantitatives,
- des moyens d'enregistrement des données.

13. Appareil selon l'une quelconque des revendications précédentes, **caractérisé en ce qu'**il comprend en outre des moyens de traitement des données enregistrées pour chacun des échantillons au cours d'un unique cycle d'évaporation depuis l'état saturé jusqu'à l'état sec, et des moyens de calcul de la courbe de retrait et de la courbe du potentiel de l'eau desdits échantillons.

## Patentansprüche

1. Vorrichtung für die physikalische Analyse eines Bodens in Bezug auf seinen Wassergehalt, umfassend einen Probenwechsler (100), der geeignet ist, mindestens zwei Träger (1) von Bodenproben auszunehmen, wobei der Probenwechsler mit Mitteln ausgestattet ist, um jeden Probenträger und seine Probe (2) der Reihe nach und nach einem zeitlich wiederholten Zyklus in einem Messbereich anzuordnen, **dadurch gekennzeichnet, dass** sie Folgendes aufweist:
- Mittel zum Messen der Masse der Proben, umfassend eine Waage (4), die mit einer Schale (5) ausgestattet ist, die geeignet ist, den Probenträger (1) und seine Probe (2) aufzunehmen und zu wiegen;
- Mittel zum Messen der Abmessungen der Proben, umfassend ein optisches System zum Messen der Höhe und des Durchmessers der Probe, wenn diese auf der Waagschale angeordnet ist; und
- Mittel zum Messen des Wasserpotentials der Proben, die für jede Probe ein Tensiometer "mit poröser Kerze" aufweisen, das geeignet ist, das Wasserpotential der Probe zu messen, und einen biegsamen Schlauch (6) aufweisen, der durch eine Kerze (7) aus durchlässiger Keramik an einem Ende und ein Manometer (8) an dem anderen Ende geschlossen ist, wobei das Manometer in dem Träger der Probe angeordnet ist;
wobei alle Mittel zum Messen in dem Messbereich gruppiert sind, um gleichzeitig an einer bestimmten Probe eingesetzt zu werden, wenn sie in dem Messbereich angeordnet ist.

2. Vorrichtung nach Anspruch 1, **dadurch gekennzeichnet, dass** der Probenwechsler eine kreisförmige Platte (3) aufweist, die mit mindestens zwei Durchgangsbohrungen (30) versehen ist, die jeweils geeignet sind, in Anlage an ihrem Rand einen im Wesentlichen zylindrischen Probenträger (1) aufzunehmen, wobei die Platte in Drehung und in vertikaler Verschiebung derart beweglich ist, dass, wenn die Platte abgesenkt wird, einer der Probenträger (1) auf der Waage (4) mit seiner Probe (2) in dem Messbereich angeordnet wird.

3. Vorrichtung nach einem der Ansprüche 1 oder 2, **dadurch gekennzeichnet, dass** jeder Probenträger (1) Folgendes aufweist:
- einen im Wesentlichen zylindrischen Sockel (9), der von einer horizontalen Platte (10) überragt ist, die dazu bestimmt ist, eine Probe (2) zu tragen, und
- einen Manometerblock (11), der an der Wand des Sockels befestigt ist und in dem eine hermetische Kammer (12) angeordnet ist, die mit der porösen Kerze (7) über den biegsamen Schlauch (6) verbunden ist, wobei die Kammer mit einem Membrandrucksensor (13) verbunden ist.

4. Vorrichtung nach dem vorhergehenden Anspruch, **dadurch gekennzeichnet, dass** jeder Sensor (13) einen Puffer zum Verschließen der Kammer (12) bildet.

5. Vorrichtung nach einem der Ansprüche 3 oder 4, **dadurch gekennzeichnet, dass** jede Kammer (12) eine gewölbte Decke (14) aufweist, an deren Spitze eine Füllleitung (15) mündet, die mit einem Ventil (16) versehen ist.

6. Vorrichtung nach einem der Ansprüche 1 bis 5, **dadurch gekennzeichnet, dass** jedes Manometer (8) mit elektrischen Schützen (17) verbunden ist, die in der Nähe der Basis des Sockels (9) gehalten sind, und die Schale (5) der Waage (4) mit einer Platte (18) versehen ist, die mit elektrischen Kontaktplättchen (19) ausgestattet ist, wobei die Schütze derart angeordnet sind, dass sie mit den Korttaktplättchen in Kontakt zu treten, wenn der Probenträger (1) auf der Waage angeordnet ist.

7. Vorrichtung nach einem der Ansprüche 3 bis 6, **dadurch gekennzeichnet, dass** die Platte (10) von jedem Probenträger (1) mit Perforationen ausgestattet ist, die den Durchgang von Wasser und Luft zwischen der Atmosphäre und der Basis der Probe (2) ermöglichen.

8. Vorrichtung nach einem der Ansprüche 1 bis 7, **dadurch gekennzeichnet, dass** das optische System zum Messen des Durchmessers der Probe (2) zwei Laser-Lichtschrankensensoren (20', 20") aufweist, die in dem Messbereich auf beiden Seiten der Probe, wenn diese auf der Waage (4) angeordnet ist, in einem gegenseitigen Abstand angeordnet sind, der kleiner als der angenommene Mindestdurchmesser der Proben ist.

9. Vorrichtung nach einem der Ansprüche 1 bis 8, **dadurch gekennzeichnet, dass** das optische System zum Messen der Höhe der Probe (2) einen berührungslosen Laser-Sensor (21) aufweist, der senkrecht über dem Messbereich angeordnet ist, der geeignet ist, einen Lichtstrahl auf ein Plättchen (23) zu emittieren, das flach auf der Probe angeordnet ist, und den reflektierten Strahl zu erfassen, um durch Triangulation die Entfernung des Plättchens zu bestimmen.

10. Vorrichtung nach einem der Ansprüche 1 bis 9, **dadurch gekennzeichnet, dass** die Waage (4) eine Vorrichtung zum Höhenausgleich in Abhängigkeit von der Masse, die auf ihrer Schale (5) angeordnet ist, aufweist.

11. Vorrichtung nach einem der vorhergehenden Ansprüche, **dadurch gekennzeichnet, dass** der Probenwechsler (100) eine kreisförmige Platte (3), die acht Durchgangsbohrungen (30) aufweist, und eine Hubsäule (24) aufweist, die von einem ersten Motor, der die vertikale Verschiebung der kreisförmigen Platte gewährleistet, und von einem zweiten Motor betätigt ist, der die Drehung der kreisförmigen Platte gewährleistet.

12. Vorrichtung nach einem der vorhergehenden Ansprüche, **dadurch gekennzeichnet, dass** sie ferner Folgendes aufweist:
- einen thermostatisierten Trockenschrank, der geeignet ist, mindestens den Probenwechsler und alle Mittel zum Messen zu enthalten,
- Mittel zum Steuern des Probenwechslers und der Mittel zum Messen,
- Mittel zum Empfangen und Verarbeiten der gemessenen Signale, um quantitative Messdaten zu erhalten,
- Mittel zum Speichern der Daten.

13. Vorrichtung nach einem der vorhergehenden Ansprüche, **dadurch gekennzeichnet, dass** sie ferner Mittel zum Verarbeiten der Daten, die für jede der Proben im Laufe eines einzelnen Verdunstungszyklus von dem gesättigten Zustand bis zu dem trockenen Zustand gespeichert sind, und Mittel zum Berechnen der Rückstellkurve und der Kurve des Wasserpotentials der Proben aufweist.

## Claims

1. Apparatus for physically analyzing soil with regard to its hydrological state, comprising a sample changer (100) that can receive at least two soil sample holders (1), said sample changer being equipped with means for placing each sample holder and its sample (2) in a measurement area in turns and according to a cycle repeated over time, **characterized in that** it comprises:
- means of measuring the mass of said samples, comprising a balance (4) with a pan (5) able to receive and weigh the sample holder (1) and its sample (2);
- means of measuring dimensions of said samples, comprising an optical system for measuring the height and diameter of said sample when it is placed on said weighing pan;
- means of measuring the water potential of said samples, comprising for each sample, a tensiometer known as a "porous cup tensiometer" able to measure the water potential of said sample, comprising a flexible tube (6) closed by a cup (7) made of porous ceramic at one extremity and by a pressure gauge (8) at the other, said pressure gauge being housed in said sample's holder;
all said measurement means being grouped in said measurement area to be utilized at the same time on a given sample when this sample is placed in said measurement area.

2. Apparatus according to claim 1, **characterized in that** the sample changer comprises a circular tray (3) having at least two through holes (30), each able to receive, supported on its edge, a substantially cylindrical sample holder (1), said tray being mobile in rotation and in vertical translation, such that when the tray is lowered, one of the sample holders (1) is placed on the balance (4) with its sample (2) in the measurement area.

3. Apparatus according to any of claims 1 or 2, **characterized in that** each sample holder (1) comprises:
- a substantially cylindrical base (9) with a horizontal plate (10), designed to support a sample (2), mounted above it, and
- a pressure gauge unit (11) fixed to the wall of said base, and wherein a hermetic chamber (12) is formed communicating with the porous cup (7) by means of the flexible tube (6), said chamber being associated with a membrane pressure sensor (13).

4. Apparatus according to the preceding claim, **characterized in that** each sensor (13) forms a plug sealing the chamber (12).

5. Apparatus according to any of claims 3 or 4, **characterized in that** each chamber (12) comprises a curved ceiling (14) at the top of which a filler line (15) fitted with a valve (16) emerges.

6. Apparatus according to any of claims 1 to 5, **characterized in that** each pressure gauge (8) is connected to electrical contactors (17) held close to the bottom of the base (9), and the pan (5) of the balance (4) is equipped with a board (18) equipped with electrical terminals (19), said contactors being arranged so as to come into contact with said terminals when the sample holder (1) is placed on the balance.

7. Apparatus according to any of claims 3 to 6, **characterized in that** the plate (10) of each sample holder (1) is fitted with perforations permitting the water and air to pass between the atmosphere and the bottom of the sample (2).

8. Apparatus according to any of claims 1 to 7, **characterized in that** the optical system for measuring the diameter of the sample (2) comprises two laser barrier sensors (20', 20") placed in the measurement area either side of said sample when it is placed on the balance (4), at a mutual distance less than the assumed minimum diameter of the samples.

9. Apparatus according to any of claims 1 to 8, **characterized in that** the optical system for measuring the height of the sample (2) comprises a contactless laser sensor (21) placed above the measurement area, able to emit a light beam towards a disk (23) that is placed flat on said sample and to detect the reflected beam to determine by triangulation how far away said disk is.

10. Apparatus according to any of claims 1 to 9, **characterized in that** the balance (4) comprises a device for compensating the height according to the mass placed on its pan (5).

11. Apparatus according to any one of the preceding claims, **characterized in that** the sample changer (100) comprises a circular tray (3) comprising eight through holes (30), and a lifting column (24) actioned by a first motor vertically moving said circular tray and by a second motor rotating said circular tray.

12. Apparatus according to any one of the preceding claims, **characterized in that** it comprises, in addition:
- a temperature-controlled oven able to contain at least the sample changer and all of the measurement means,
- means of controlling the sample changer and the measurement means,
- means of receiving and processing measured signals to obtain quantitative measurement data,
- means of recording the data elements.

13. Apparatus according to any one of the preceding claims, **characterized in that** it comprises, in addition, means of processing data recorded for each of the samples during a single evaporation cycle from the saturated state through to the dry state, and means of calculating the shrinkage curve and the water potential curve for said samples.
